# DEMANDE DE BREVET EUROPEEN

(11) **EP 1 398 035 A1**
(43) Date de publication de la demande: **17.03.2004**
(21) Numéro de dépôt: 03292235.3
(22) Date de dépôt: 11.09.2003
(51) Int. Cl.: A61K 35/78, A61K 7/48

(54) **Application locale cutanée d'émulsions caractérisées par leurs propriétés antiprostanoidiques**

(30) Priorité: 12.09.2002 FR 0211291; 10.09.2003 FR 0310668
(71) Demandeur: Morelle, Jean, 75010 Paris (FR); Lauzanne, Eliane, 75010 Paris (FR); ROTHFUSS, Jacqueline, 67380 Lauterbourg (FR)
(72) Inventeur: Morelle, Jean, 75010 Paris (FR); Lauzanne, Eliane, 75010 Paris (FR); ROTHFUSS, Jacqueline, 67380 Lauterbourg (FR)

(57) **Abrégé**

L'invention concerne l'application locale cutanée d'émulsions, caractérisées par la présence d'extraits de légumes et de fruits responsables simultanément d'une action inhibitrice vis-à-vis des enzymes oxydantes générant les prostanoïdes et d'une action destructrice des hydroxyperoxydes prostanoïdiques

## Description

Il est universellement admis, que les prostanoïdes sont des acides gras caractérisés par une structure comportant des fonctions hydroxyles stables ou instables, issus de la peroxydation de l'acide arachidonique, principal acide gras polyinsaturé de la membrane cellulaire. Ces prostanoïdes sont dus à l'action de systèmes enzymatiques oxydants endogènes telles que, les lipoxygénases, les cyclolipoxygénases ou la xanthine -oxydase. Ce sont des produits nocifs pour l'organisme, conduisant à un vieillissement prématuré, et au déséquilibre métabolique. Parmi ces différents dérivés hydroxylés, citons : L'acide hydroxy-15-éicosatétraène-5-8-11-13-oïque ou 15 HETE (SERHAN C- HAMBERG H- SAMUELSSON B-Biochem. Biophys. Res. Commun. 1984 118 943) par exemple.

Ces substances peuvent provoquer des dysfonctionnements locaux en interférant sur diverses molécules biologiques, donnant lieu à la formation de métabolites toxiques ; ceci en cas de déficience en protecteurs antioxydants ; c'est ce qui est constaté avec l'augmentation de l'âge : Les antioxydants naturels diminuent, les enzymes protectrices perdent leur efficacité, assurant ainsi moins de protection face aux mécanismes de formation des hydroperoxydes prostanoïdiques endogènes.
De plus, il a été largement démontré que la dégradation ultime des acides prostanoïdiques conduit à la formation de malondialdéhyde (MDA), aldéhyde particulièrement réactif avec les protéines.
La formation des prostanoïdes et de leurs métabolites, en dehors du facteur alimentaire est issue des mécanismes oxydatifs se réalisant dans les cas suivants :

### 1-L'effort physique

Celui-ci provoque un stress oxydatif, caractérisé par la présence de malondialdéhyde et de fer dans la sueur [BRADY et Col. J. of animal Sci- 1978 *47* 492- WILBUR et Col. Arch. Of Biochem. 1949 *24* 30- SJODIN et Col. Sports Med. 1990 *10* (4) 236)]

### 2-L'altération visuelle (lumière, écran d'ordinateurs, de télévision)

La rétine est particulièrement riche en acides gras polyinsaturés pouvant générer des lipoperoxydes oxydant le glutathion réduit ( BABIZHAEV M- Vior.Led. Khim. 1985 *31* (6) 100)

### 3- L'agression thermique ( brûlures locales)

Dans ce cas, on constate la formation de prostanoïdes et de leurs métabolites : Les leucotriènes, éminemment toxiques et de l'important marqueur de la dégradation oxydative : Le malondialdéhyde ( MDA) De plus, l'activation des polynucléaires neutrophiles génère des radicaux libres comme l'oxygène singulet par exemple, ( YAGI K- lipid peroxides in Biol. Academic Press 1982)

### 4-La dégradation oxydative du cartilage articulaire

Elle est caractérisée par une importante dégradation de l'acide arachidonique,exprimée par la formation de MDA. Il est reconnu, que les oxygènes actifs sont responsables des dommages du cartilage (HENROTIN et Col . Experientia Suppl. 1992 62 308)

Etant donné, que les substances antioxydantes proviennent essentiellement de l'alimentation, que dans de nombreux cas, notre alimentation est oxydative plutôt qu'antioxydative, que de plus, les antioxydants alimentaires, principalement les polyphénols des légumes et des fruits ne sont pas toujours en mesure de neutraliser la peroxydation locale, que ce soit au niveau musculaire, oculaire, cartilagineux ou du tissu brûlé.
La présente invention a pour objet de lutter contre l'activité nocive locale des prostanoïdes et de leurs métabolites, par application cutanée d'émulsions comportant de 4 à 10% d'un extrait aqueux titré, de légumes ou de fruits, contenant des polyphénols, doué d'une double activité :
- La première, permettant d'inhiber l'action des enzymes endogènes oxydantes locales: (xanthine- oxydase, lipoxygénases, cyclolipoxygénases ) empêchant ainsi la formation des hydroperoxydes prostanoïdiques et de leurs métabolites.
- La seconde, de neutraliser les hydroperoxydes prostanoïdiques pouvant localement exister.

On voit tout l'intérêt que peut présenter l'utilisation locale de telles émulsions ; elles permettent d'apporter directement sur les parties concernées des quantités d'extraits conduisant à inhiber l'action des enzymes oxydantes ou à neutraliser les prostanoïdes et leurs métabolites, et de ce fait, à empêcher les effets nocifs suscités par ce type de molécules, complétant ainsi un régime alimentaire déficient en polyphénols ou ne permettant pas d'atteindre les zones concernées, que ce soit au niveau musculaire, cartilagineux, oculaire ou cutané

Dans le cadre de la présente invention on utilisera préférentiellement des extraits aqueux de légumes ou de fruits, doués de cette double action, ayant une grande capacité à inhiber l'activité des enzymes oxydantes.
Cette capacité est mesurée in vitro par la non formation d'oxygène actif ; ainsi dans le cas de la xanthine-oxydase , on trouve :
- Acide oléique témoin = 40µg/g d'oxygène actif
- Acide oléique témoin+xanthine-oxydase= 200µg/g d'oxygène actif
- Acide oléique témoin+xanthine-oxydase+extrait d'ortie= 0 µg/g d'oxygène actif
- Acide oléique témoin+xanthine-oxydase+extrait d'endive= 12µg/g d'oxygène actif

Ces résultats sont confirmés in vivo, par la viabilité des mitochondries cellulaires.
L'utilisation de ces légumes ou fruits, consommés dans le cadre alimentaire, évite toute suspicion sur d'éventuels effets nocifs ou sur leurs propriétés pharmacologiques.

### Parmi les légumes et les fruits on aura recours à titre d'exemples non limitatifs :

***A un extrait d'ortie,*** dont 1g est capable de capter de 2000 à 4000 microgrammes (µg) d'oxygène actif. L'introduction dans une émulsion d'un tel extrait à raison de 5% conduit à une destruction de 100µg à 200µg d'oxygène actif par gramme d'émulsion et inhibe totalement l'action enzymatique oxydante.

***A un extrait du chou brocoli ou d'orange,*** permettant en moyenne la destruction de 1600µg d'oxygène actif pour un gramme d'extrait. L'introduction de ces extraits dans une émulsion à raison de 5% permettra une destruction de 80 µg d'oxygène actif pour 1 gramme d'émulsion et inhibe l'activité enzymatique

***A des extraits d'endive ou de fraise,*** d'une capacité antioxygène actif de 1200µg/g ; 5% de ces extraits dans une émulsion conduiront à 60µg d'oxygène actif détruit, et comme dans les précédents exemples à une inactivation enzymatique.

Selon l'invention, le minimum d'oxygène actif neutralisé par gramme d'émulsion est de 50µg Chaque extrait faisant l'objet d'une mesure déterminant sa capacité anti-enzymatique.

| **A titre d'exemples non limitatifs d'émulsions destinées à une application cutanée locale :** | g | |
|---|---|---|
| 1 - Acide stéarique | 10 | |
| Stéarate de polyéthylèneglycol | 5 | |
| Propylèneglycol | 10 | |
| Extrait d'ortie 2000µg0₂g * | 4 | * nombre de µg d'O₂ détruit par gramme d'extrait |
| Paraoxybenzoates | 0,3 | |
| Eau qsp | 100 | |
| | | |
| 2- Excipient selon 1 | | |
| Extrait de melon 1500µg0₂/g | 5g | |
| | | |
| 3-Excipient selon 1 | | |
| Extrait de chou brocoli 1200µg0₂/g | 6g | |
| | | |
| 4-Excipient selon 1 | | |
| Extrait de poivron vert 800µg0₂/g | 9g | |
| | | |
| 5-Excipient selon 1 | | |
| Extrait de carotte 700µg0₂/g | 10g | |

## Revendications

1. Compositions comportant des extraits aqueux de légumes et de fruits alimentaires, **caractérisées en ce qu'**elles sont utilisées en application cutanée locale, sous la forme d'émulsions, étant douées d'une double activité :
La première étant d'inhiber l'action des enzymes oxydantes locales, telles que la xanthine-oxydase, les lipoxygénases, et les cyclolipoxygénases responsables de la formation des prostanoïdes.
La seconde étant de neutraliser les hydroperoxydes prostanoïdiques pouvant exister.

2. Compositions selon la revendication 1, **caractérisée en ce que** les émulsions comportent de 4 à 10% d'un extrait aqueux titré, de légume ou de fruit contenant des polyphénols, choisi en fonction de sa capacité à inhiber l'action des enzymes oxydantes locales et en fonction de sa capacité à neutraliser les prostanoïdes formés.

3. Compositions selon les revendications 1 et 2 **caractérisées en ce que** le minimum d'oxygène actif neutralisé par gramme d'émulsion est de 50µg

4. Compositions selon les revendications de 1 à 3, **caractérisées en ce qu'**elles sont appliquées localement pour apporter directement sur les parties concernées les quantités d'inhibiteurs enzymatiques, empêchant la formation d'oxygène nocif, ou neutralisant les hydroperoxydes prostanoïdiques existant ; que ce soit au niveau musculaire,oculaire, cartilagineux ou cutané.
